# EUROPÄISCHE PATENTSCHRIFT

(11) **EP 2 341 884 B1**
(45) Veröffentlichungstag und Bekanntmachung des Hinweises auf die Patenterteilung: **23.12.2015**
(21) Anmeldenummer: 09748021.4
(22) Anmeldetag: 06.10.2009
(51) Int. Cl.: A61J 1/10, G01K 3/04, A61J 1/14

(54) **Behälter mit Computerprodukt**
Container having a computer product
Récipient avec produit formant ordinateur

(30) Priorität: 22.10.2008 DE 102008052693
(43) Veröffentlichungstag der Anmeldung: 13.07.2011
(73) Patentinhaber: Sartorius Stedim Biotech GmbH, 37079 Göttingen (DE)
(72) Erfinder: BAUMFALK, Reinhard, 37083 Göttingen (DE); KAHLERT, Wolfgang, 34327 Körle (DE); LÜDERS, Julia, 31246 Lahstedt (DE); RIECHERS, Daniel, 30451 Hannover (DE)
(74) Vertreter: Müller-Boré & Partner Patentanwälte PartG mbB
(86) Internationale Anmeldenummer: PCT/EP2009/007171
(87) Internationale Veröffentlichungsnummer: WO 2010/046029

(56) Entgegenhaltungen:
- EP-A2- 1 319 385
- WO-A1-00/42969
- WO-A1-2004/008387
- AT-B- 403 245
- DE-U1- 20 106 542

## Beschreibung

Die vorliegende Erfindung betrifft ein Behältnissystem, eine Verwendung eines Behältnissystems sowie ein Verfahren zur Herstellung eines Behältnissystems.

Für nicht klinische experimentelle Prüfungen von Stoffen oder Zubereitungen, deren Ergebnisse die behördliche Bewertung möglicher Gefahren für Mensch und Umwelt ermöglichen sollen, wird die Einhaltung der "Gute Laborpraxis"-(GLP-)Grundsätze im Chemikaliengesetz verbindlich vorgeschrieben. In der ChemVwV-GLP wird der Anwendungsbereich für Chemikalien, Pflanzenschutzmittel, Arzneimittel, Sprengstoffe, Stoffe im Gefahrguttransport und Lebensmittel- und Futtermittelzusatzstoffe konkretisiert.

Um eine sichere und vollständige Dokumentation eines manuellen Prozeßschrittes in einer Versuchsreihe, insbesondere GLP-konform, zu gewährleisten, ist der durchführende Benutzer (Mitarbeiter) angewiesen, die Dokumentation der relevanten Prozeßparameter sofort nach dem Ausführen der Handlung vorzunehmen. Dies wird heute nach wie vor in erster Linie über eine papiergebundene Aufzeichnung vollzogen, da dies einfach und schnell in den Ablauf der Versuchsreihe zu integrieren ist. Allerdings erfolgt die langfristig Dokumentation der Prozeßparameter in der Regel digital, so daß die dokumentierten Daten nachträglich digitalisiert werden müssen. Dieser Schritt der Datenmigration ist zeitaufwendig und unterliegt einer zusätzlichen Kontrolle, ob die Daten korrekt übertragen wurden. Alternativ könnten die erfaßten Prozeßparameter und Durchführungsbestätigungen direkt in einem elektronischen Erfassungssystem eingegeben werden, was aufgrund der zusätzlichen Wege für die Benutzer bei fest installierten Erfassungssystemen nur ungern angenommen wird und schwer in den Prozeßablauf einzubinden ist.

Die WO 2006/040106 A1 offenbart eine in sich geschlossene Einweganalysevorrichtung zur qualitativen und quantitativen Untersuchung von Proben. Die Einweganalysevorrichtung umfaßt einen Sensor zur Messung einer chemischen oder physikalischen Eigenschaft einer Substanz und eine Datenerfassungsvorrichtung mit einem Datenspeicher, wobei der Sensor und die Datenerfassungsvorrichtung integrale Bestandteile einer Verpackung aus einem blattähnlichen, bedruckbaren und faltbaren Material sind, die alle Bestandteile umschließt und schützt.

Die WO 2006/086489 A1 offenbart ein Kontrollsystem für einen Bioreaktor mit einer Kontrolleinheit, welche ausgelegt ist, Informationen über die Bedingungen bzw. Zustände in dem Bioreaktor zu erhalten und die Prozesse in dem Bioreaktor zu steuern.

Die WO 00/42969 A1 offenbart eine Vorrichtung zur Überwachung der Qualität und zur Identifikation von einer in einem Behälter aufbewahrten Flüssigkeit. Dazu ist an dem Behälter eine integrierte Schaltung angeordnet, die mit Sensoren verbunden ist und die von den Sensoren erfaßten Meßwerte mittels einer Prozessoreinheit speichert bzw. an einen externen Rechner überträgt.

Die Druckschrift AT 403 245 B offenbart einen flexiblen, beutelförmigen Behälter zur Aufbewahrung von Blut, Blutplasma bzw. Injektionslösungen mit einer zumindest auf ihrer einen Seite mit dem Behälterinneren in Berührung stehenden Tasche, die einen Schaltungsträger enthält, auf dem eine Stromversorgung, ein Temperatursensor und eine Verarbeitungslogik angeordnet sind, wobei der Temperatursensor das Behälterinnere thermisch kontaktiert und wobei die Verarbeitungslogik zumindest die vom Temperatursensor abgegebenen Daten verarbeitet.

Die Druckschrift WO 2004/008 387 A1 offenbart ein System zur Überwachung von medizinischen Geräten umfassend einen RFID-Tag. Das RFID-Tag enthält Informationen über das Gerät, wie zum Beispiel Daten über die Herstellung, den Vertrieb und Verkauf. Das System enthält ferner eine Lesevorrichtung, welcher den RFID-Tag auslesen kann um eine Datenbank zu aktualisieren. Die Informationen in dem RFID-Tag können mittels der Lesevorrichtung überarbeitet werden.

Die Druckschrift EP 1 319 385 A2 offenbart einen Behälter mit einer äußeren Abdeckung, einem Innenraum für Blut oder Biutprodukte und einem Raum, der innerhalb der äußeren Abdeckung angeordnet ist und gegenüber dem Innenraum abgedichtet ist, wobei in dem Raum eine Temperaturmeßsonde angeordnet ist.

Die Druckschrift DE 201 06 542 U1 offenbart ein Identifikationselement für Gegenstände, bei dem auf einem flächigen Träger mindestens ein Halbleiter-Chip mit Speicherfunktion mit einer auf dem Träger ausgebildeten Leiterbahnstruktur und mindestens einem elektrochemischen Zellaufbau, als Elektroenergiespeicher verbunden ist, wobei die Leiterbahnstruktur als Antenne zur drahtlosen Informationsübertragung mit dem Halbleiter-Chip, der mindestens mit einem Sensor verbunden oder in den mindestens ein Sensor zur Erfassung physikalischer Größen integriert ist, ausgebildet ist.

Es ist eine Aufgabe der vorliegenden Erfindung, die Erfassung und Dokumentation von relevanten Prozeßparametern über die gesamte Dauer einer Versuchsreihe zu vereinfachen und die Sicherheit der Dokumentation zu erhöhen. Diese Aufgabe wird durch ein Behältnissystem mit den Merkmalen des Anspruchs 1, eine Verwendung des Behältnissystem gemäß Anspruch 12 und ein Verfahren mit den in Anspruch 13 definierten Schritten gelöst. Bevorzugte Ausführungsformen sind Gegenstand der abhängigen Ansprüche.

Ein Aspekt der vorliegenden Erfindung betrifft ein Behältnissystem, insbesondere ein Aufbewahrungs- und/oder Reaktionsbehältnissystem für Flüssigkeiten, umfassend:
- einen Behälter und
- eine an dem Behälter befestigte elektronische Dokumentationsvorrichtung, wobei die Dokumentationsvorrichtung weiter umfaßt:
   -- eine Zeitgebereinrichtung zum Erfassen eines Zeitpunkts,
   -- zumindest eine Sensorvorrichtung,
   -- zumindest eine Eingabevorrichtung, die ausgelegt ist, um eine manuelle Betätigung zu erfassen,
   -- eine Speichereinrichtung, welche ausgelegt ist, zumindest einen von der zumindest einen Sensorvorrichtung erfaßten Meßwert und/oder eine von der zumindest einen Eingabevorrichtung erfaßte Eingabe auslesbar zu speichern und
   -- zumindest eine Anzeigevorrichtung (27; 29), um einem Benutzer einen Zustand der Dokumentationsvorrichtung (5) und/oder einen in der Speichereinrichtung (17) gespeicherten Inhalt anzuzeigen,
wobei dem zumindest einen Meßwert und/oder der zumindest einen Eingabe ein dessen/deren Erfassung entsprechender Zeitpunkt mittels der Zeitgebereinrichtung zugeordnet ist.

Die Dokumentationsvorrichtung ist an dem Behälter angeordnet und daran befestigt, so daß eine einfache Zuordnung der von der Dokumentationsvorrichtung dokumentieren Meßwerte und/oder Eingaben (allgemein Prozeßparameter) zu dem zugehörigen Behälter ermöglicht ist. Weiter vorteilhafterweise können die Prozeßparameter direkt im Anschluß an einen Prozeßschritt von dem durchführenden Benutzer an der behandelten Probe bzw. deren Behälter dokumentiert werden. Dieser Prozeßschritt kann umfassen: eine visuelle Inspektion, ein Befüllen, ein Entleeren, ein Auslösen eines chemischen, biologischen und/oder physikalischen Vorgangs, etc. Weiter vorteilhafterweise wird neben einem Prozeßparameter ebenfalls die Erfassungszeit in der Dokumentationsvorrichtung gespeichert, so daß während oder nach der Durchführung einer Versuchsreihe alle erfaßten Prozeßparameter als Zeitreihe vorliegen und gegebenenfalls als Zeitreihe ausgewertet werden können, wodurch eine nachträgliche Aufbereitung der erfaßten Prozeßparameter zu Auswertezwecken erleichtert wird.

Ein Behälter im Sinne der Erfindung zeichnet sich dadurch aus, daß der Behälter im Hinblick auf den zu beinhaltenden Inhalt des Behälters hinreichend dicht ausgelegt ist, so daß sich der Inhalt des Behälters (oder Teile davon) nicht bestimmungswidrig über die geometrischen Grenzen des Behälters hinaus verlagern kann. Der Begriff "hinreichend dicht" im Sinne der Erfindung kann insbesondere bedeuten, daß der Behälter partikeldicht ist, vorzugsweise für Partikel mit einem Partikeldurchmesser von größer als etwa 1 mm, weiter vorzugsweise für Partikel mit einem Partikeldurchmesser von größer als etwa 100 µm, 50 µm, 10 µm, besonders bevorzugt für Partikel mit einem Partikeldurchmesser von größer als etwa 5 µm, 2 µm, 1 µm. Weiter vorzugsweise kann der Begriff "hinreichend dicht" im Sinne der Erfindung bedeuten, daß der Behälter im wesentlichen wasserdicht, feuchtigkeitsdicht, wasserdampfdicht, luftdicht, gasdicht, schutzgasdicht, inertgasdicht und/oder fluiddicht (insbesondere fluiddicht für Schutzgase wie Helium, Argon, Stickstoff, Kohlendioxid, usw.) ist, so daß das Innere des Behälters im wesentlichen vollständig wasser-, feuchtigkeits-, wasserdampf-, luft-, gas- und/oder fluiddicht von der Umgebung abgeschlossen ist.

Der Begriff "im wesentlichen dicht" kann eine geringfügige Abweichung von der vollständigen Dichtigkeit beschreiben, insbesondere eine geringe Undichtigkeit bzw. eine Leckrate von weniger als etwa 30 %, weniger als etwa 20 %, weniger als etwa 10 %, weniger als etwa 5 %, weniger als etwa 2 %, bevorzugt weniger als etwa 1 % des Inhalts des Behälters pro Jahr beinhalten. Der Begriff "im wesentlichen" umfaßt den Begriff "vollständig" bzw. "identisch", d.h. ohne Undichtigkeit bzw. mit einer Leckrate von etwa 0 %.

Die Zeitgebereinrichtung stellt insbesondere ein Datum, Uhrzeit, einen Takt und/oder einen Zählerstand etc. bereit, so daß dem von der Zeitgebereinrichtung bereitgestellten Wert bzw. dem von der Sensorvorrichtung erfaßten Meßwert bzw. der von der Eingabevorrichtung erfaßten Eingabe eindeutig ein Zeitpunkt zugeordnet werden kann.

Die Speichereinrichtung ist dazu ausgelegt Daten, wie die Erfassungszeit, einen Meßwert und/oder eine Eingabe, auslesbar zu speichern. Dem zumindest einen Meßwert bzw. der zumindest einen Eingabe ist dabei ein Zeitpunkt der Erfassung bzw. eine Erfassungszeit eindeutig zuordenbar bzw. zuzuordnen. Insbesondere ist der Zeitpunkt der Erfassung zusammen mit einem Meßwert und/oder einer Eingabe auslesbar gespeichert. Insbesondere kann auch eine eindeutige Identifikationsnummer des Behältnissystems bzw. eine Chargennummer und/oder Probennummer des Inhaltes des Behälters durch die Speichereinrichtung gespeichert sein. Dabei kann das Auslesen der Daten durch die Dokumentationsvorrichtung erfolgen und/oder durch eine externe Vorrichtung, wobei das Auslesen vor, während und/oder nach dem betriebsmäßigen Gebrauch des Behältnissystems erfolgen kann. Insbesondere kann die Speicherung der Daten in der Speichereinrichtung dauerhaft bzw. permanent und/oder unveränderlich erfolgen. In diesem Zusammenhang bedeutet "dauerhaft" bzw. "permanent", daß die gespeicherten Daten ohne Energiezufuhr von außen in der Speichereinrichtung auslesbar erhalten bleiben, d.h. daß die gespeicherten Daten nicht lediglich flüchtig gespeichert sind. "Unveränderlich" bedeutet in Verbindung mit in der Speichereinrichtung gespeicherten Daten, daß die Daten nach dem Speichervorgang nicht geändert und/oder gelöscht werden können. Insbesondere kann die Speichereinrichtung ein EPROM, ein EEPROM oder einen Flash-Speicher umfassen. Vorteilhafterweise kann durch die Speichereinrichtung das Behältnissystem und/oder dessen Inhalt eindeutig identifiziert werden, wobei ein vollständiges chronologisches Protokoll der Zustände des Behältnissystems bzw. des Inhalts bereitgestellt werden.

Vorzugsweise besteht der Behälter aus einem flexiblen Kunststoff. Flexibel bedeutet in diesem Zusammenhang, daß der Kunststoff biegsam, bevorzugt rückstellfähig biegsam, ist. Insbesondere kann der Behälter zumindest teilweise bzw. bereichsweise aus Polyethylen, Polyvinylchlorid oder Polycarbonat ausgebildet sein. Vorteilhafterweise sind Behälter aus einem dieser Kunststoffe einfach herzustellen und gegen eine Vielzahl von Substanzen chemisch inert.

Vorzugsweise ist der Behälter ein Einwegbehälter, besonders bevorzugt ein sterilisierbarer Einwegbehälter. Weiter bevorzugt ist der Behälter ausgelegt, durch Autoklavieren sterilisierbar zu sein, d.h. der Behälter ist ausgelegt, bei Temperaturen bis etwa 100 °C, bevorzugt bis etwa 120 °C, weiter bevorzugt bis etwa 150 °C im wesentlichen formstabil zu sein. Besonders bevorzugt ist der Behälter ausgelegt, durch Sterilisationsmittel sterilisierbar zu sein, insbesondere durch Ethylenoxid und/oder Ethylalkohol, d.h. das Behältermaterial ist insbesondere nicht durch Ethylalkohol lösbar. Weiter vorzugsweise ist der Behälter ausgelegt, durch gamma-Strahlung sterilisierbar zu sein.

Der Begriff "im wesentlichen" mit Bezug auf die Formstabilität kann eine geringfügige Abweichung von einer Sollform nach der Temperaturbeaufschlagung beschreiben, insbesondere eine Abweichung im Rahmen der Herstellungsgenauigkeit und/oder im Rahmen der notwendigen Genauigkeit, so daß ein Effekt beibehalten wird, wie er bei der Sollform vorhanden ist. Der Begriff "im wesentlichen" kann daher insbesondere eine Abweichung von weniger als etwa 30 %, weniger als etwa 20 %, weniger als etwa 10 %, weniger als etwa 5 %, weniger als etwa 2 %, bevorzugt weniger als etwa 1 % von einer Sollform beinhalten. Der Begriff "im wesentlichen" umfaßt den Begriff "identisch", d.h. ohne Abweichung von einer Sollform.

Die zumindest eine Eingabevorrichtung ist ausgelegt, eine manuelle Betätigung zu erfassen. Eine manuelle Betätigung umfaßt insbesondere das kraftlose Berühren, das Aufbringen einer Kraft oder das räumliche Verlagern der Eingabevorrichtung. Insbesondere kann die zumindest eine Eingabevorrichtung einen Photowiderstand oder elektrischen Leitfähigkeitssensor umfassen, um ein Berühren der Eingabevorrichtung, insbesondere mit einem Finger, zu detektieren. Weiter bevorzugt kann die zumindest eine Eingabevorrichtung zumindest einen Lagesensor umfassen, insbesondere einen Quecksilberschalter und/oder einen Trägheitssensor, der ausgelegt ist, eine räumliche Drehung und/oder Translation der Eingabevorrichtung zu detektieren.

Vorzugsweise umfaßt die zumindest eine Eingabevorrichtung, die ausgelegt ist, eine manuelle Betätigung zu erfassen, zumindest einen Taster und/oder zumindest einen Schalter, der/die bevorzugt Teil der Dokumentationsvorrichtung ist/sind. Insbesondere umfaßt die zumindest eine Eingabevorrichtung eine Tastatur, die ausgelegt ist, numerische oder alphanumerische Eingaben von Benutzern zu erfassen. Vorteilhafterweise sind manuelle Eingaben binärer Art, also Bestätigungen - beispielsweise einer fälligen visuellen Inspektion - durch das Bereitstellen eines Tasters und/oder Schalters an der Eingabevorrichtung besonders einfach durchführbar.

Vorzugsweise umfaßt das Behältnissystem eine Vielzahl von Eingabevorrichtungen, wobei die Eingabevorrichtungen eine Empfangsvorrichtung umfassen, die ausgelegt ist, ein Signal einer externen Vorrichtung zu erfassen. Bevorzugt ist die Empfangsvorrichtung ausgelegt, Eingaben von Benutzern über eine externe Vorrichtung zu erfassen. Insbesondere können numerische bzw. alpha-numerische Benutzereingaben mittels einer externen Tastatur und/oder eines externen Computers erfaßt, an die Empfangsvorrichtung übertragen und durch die Dokumentationsvorrichtung gespeichert werden. Die Übertragung kann beispielsweise über eine Bluetooth-, LAN-, WLAN-, Firewire-, USB- und/oder Funkverbindung stattfinden. Vorteilhafterweise können über die Empfangsvorrichtung, insbesondere über eine externe Tastatur, umfangreiche Eingaben (z. B. Beobachtungen bzw. Beschreibungen von Ereignissen) durch den Benutzer in der Dokumentationsvorrichtung in einfacher Weise erfaßt werden.

Vorzugsweise umfaßt die zumindest eine Empfangsvorrichtung ein elektromagnetisches Kopplungselement und/oder ein kapazitives Kopplungselement und/oder ein induktives Kopplungselement und/oder ein galvanisches Kopplungselement. Insbesondere kann die Empfangsvorrichtung eine Antenne (z. B. eine RFID-Antenne), eine Spule, einen Infrarotempfänger, einen elektrischen Verbinder, einen Glasfaserverbinder etc. umfassen.

Vorzugsweise weist der Behälter eine Versiegelungseinrichtung auf. Die Versiegelungseinrichtung ist bevorzugt dazu ausgelegt, das Innere des Behälters vom Äußeren zu trennen. Die Trennung ist dicht, insbesondere fluiddicht und/oder steril.

Vorzugsweise umfaßt die Versiegelungseinrichtung eine Folie und/oder zumindest einen Leiter aus Metall und/oder einem elektrisch leitfähigen Polymer. Insbesondere umfaßt die Versiegelungseinrichtung eine sterile Membran. Weiter bevorzugt umfaßt die Versiegelungseinrichtung eine Folie aus einem elektrisch nicht leitfähigen Material, in welches dünne Fäden bzw. Drähte eines elektrisch leitfähigen Materials eingebettet sind.

Vorzugsweise detektiert die zumindest eine Sensorvorrichtung den Versiegelungszustand der Versiegelungseinrichtung, wobei bevorzugt der zugeordnete Zeitpunkt der Erfassung einem Bruch der Versiegelungseinrichtung entspricht. Besonders bevorzugt detektiert die zumindest eine Sensorvorrichtung Änderungen der elektrischen Leitfähigkeit eines elektrisch leitfähigen Teils der Versiegelungseinrichtung, insbesondere einer elektrisch leitfähigen Folie und/oder von in der Versiegelungseinrichtung bzw. einer Folie eingebetteten elektrisch leitfähigen Drähten bzw. Fäden.

Weiter vorzugsweise umfaßt die Versiegelungseinrichtung ein Ventil, insbesondere ein Drehventil, und/oder einen Verschluß, insbesondere einen Drehverschluß oder einen Abreißverschluß. An dem Ventil bzw. dem Verschluß ist vorzugsweise ein elektrisch leitfähiger Bereich ausgebildet, so daß der Zustand des Ventils bzw. des Verschlusses (Offen/Geschlossen) dadurch detektiert werden kann, daß der elektrisch leitfähige Bereich des Ventils bzw. des Verschlusses einen Stromkreis schließt oder nicht.

Vorteilhafterweise kann durch die Sensorvorrichtung, welche den Versiegelungszustand der Versiegelungseinrichtung detektiert, ein Siegelbruch dokumentiert werden, beispielsweise der Anbruch eines in einer Versuchsreihe benötigten Ausgangsstoffes, einer Blutkonserve oder ähnliches. Dadurch ist jederzeit nachvollziehbar, ab welchem Zeitpunkt keine Sterilität des Inhaltes des Behälters vorlag, so daß ab diesem Zeitpunkt die Verfallszeit des Inhaltes unter offenen Lagerbedingungen zu laufen beginnt. Dadurch ist vorteilhafterweise eine Qualitätskontrolle des verwendeten Inhalts jederzeit möglich, insbesondere kann auch nach der Durchführung einer Versuchsreihe festgestellt werden, ob zu einem beliebigen Zeitpunkt der Versuchsreihe verfallene Ausgangsstoffe Verwendung fanden.

Dazu sind vorzugsweise das Herstellungsdatum, das Sterilisationsdatum, das Verfallsdatum des Beutels, das Verfallsdatum des Inhalts unter sterilen Bedingungen und/oder das Verfallsdatum des Inhalts unter nicht-sterilen Bedingungen in der Speichereinrichtung abspeicherbar bzw. abgespeichert und auslesbar.

Vorzugsweise umfaßt die zumindest eine Sensorvorrichtung einen Temperatursensor und/oder einen Photosensor und/oder einen elektrischen Widerstandssensor und/oder einen elektrischen Leitfähigkeitssensor und/oder einen pH-Sensor und/oder einen Sensor zur Bestimmung der Konzentration gelöster Gase und/oder der Konzentration von im Behälterinneren vorliegender Substanzen (beispielsweise Sauerstoff, Kohlendioxid und/oder Chlorophyll) und/oder einen Sensor zur Bestimmung einer Partikel- und/oder Zelldichte. Insbesondere ist der Photosensor ausgelegt, um eine photometrische Messung durchzuführen, beispielsweise das Bestimmen einer Trübung oder eines Farbumschlages. Besonders bevorzugt umfaßt die zumindest eine Sensorvorrichtung einen nicht-invasiven Sensor, d.h. einen Sensor, welcher die Messung eines Parameters erlaubt, ohne die Integrität des Behälters aufzuheben bzw. ohne das Innere des Behälters zu kontaktieren. Insbesondere sind die nicht-invasiven Sensoren als Einwegsensoren ausgebildet.

Vorzugsweise umfaßt die Dokumentationsvorrichtung zumindest eine Anzeigevorrichtung, um einem Benutzer einen Zustand der Dokumentationsvorrichtung und/oder einen in der Speichereinrichtung gespeicherten Inhalt anzuzeigen.

Vorzugsweise umfaßt die zumindest eine Anzeigevorrichtung zumindest eine optische Anzeige und/oder zumindest eine akustische Anzeige und/oder zumindest eine haptische Anzeige. Insbesondere umfaßt die Anzeigevorrichtung eine Leuchtdiode (insbesondere verschiedenfarbige LEDs, z. B. eine rote LED als Warnsignal) und/oder eine alpha-numerische Anzeige (Display, insbesondere LCD- oder Foliendisplay). Weiter bevorzugt umfaßt die Anzeigevorrichtung eine piezoelektrische Schallquelle und/oder einen Lautsprecher. Besonders bevorzugt umfaßt die Anzeigevorrichtung einen Vibrationsalarm. Vorteilhafterweise kann die Anzeigevorrichtung signalisieren, daß ein Prozeßparameter, beispielsweise die Temperatur bzw. eine Verletzung der Versiegelung, einen vorbestimmbaren bzw. vorbestimmten Grenzwert erreicht bzw. überschritten hat. Insbesondere in diesem Fall kann die Anzeigevorrichtung verwendet werden, um eine Bestätigung durch einen Benutzer mittels der Eingabevorrichtung anzufordern. Dadurch kann insbesondere unterschieden werden, ob eine Verletzung der Versiegelung des Behälters absichtlich oder unabsichtlich erfolgt ist.

Vorzugsweise umfaßt die Dokumentationsvorrichtung zumindest eine Sendevorrichtung, die ausgelegt ist, um einen Zustand der Dokumentationsvorrichtung und/oder einen in der Speichereinrichtung gespeicherten Inhalt an eine externe Vorrichtung zu senden. Insbesondere kann die externe Vorrichtung ein Computer sein, der ausgelegt ist, die von der Sendevorrichtung gesendeten Daten zu empfangen, zu speichern und/oder weiter zu verarbeiten. Die Übertragung von der Sendevorrichtung zu der externen Vorrichtung kann beispielsweise über eine Bluetooth-, LAN, WLAN, Firewire, USB- und/oder Funkverbindung stattfinden. Besonders vorzugsweise ist die zumindest eine Sendevorrichtung identisch zu zumindest einer Empfangsvorrichtung der Dokumentationsvorrichtung.

Vorzugsweise umfaßt die zumindest eine Sendevorrichtung ein elektromagnetisches Kopplungselement und/oder ein kapazitives Kopplungselement und/oder ein induktives Kopplungselement und/oder ein galvanisches Kopplungselement. Insbesondere kann die Sendevorrichtung eine Antenne, eine Spule, einen Infrarotempfänger, einen elektrischen Verbinder, einen Glasfaserverbinder etc. umfassen.

Vorzugsweise umfaßt die Dokumentationsvorrichtung eine Stromquelle. Die Stromquelle umfaßt insbesondere eine Stromspeichereinrichtung, beispielsweise eine Batterie, einen Akkumulator und/oder einen Kondensator. Weiter bevorzugt umfaßt die Stromquelle eine Stromerzeugereinrichtung, beispielsweise eine Solarzelle, eine Brennstoffzelle und/oder eine induktives Bauteil (z. B. eine Spule), welches aus einem das Bauteil durchfließenden, zeitlich variablen Magnetfeld einen elektrischen Strom generiert (z. B. eine Radiofrequenzantenne). Die Stromquelle ist insbesondere ausgelegt einen kontinuierlichen Betrieb der Dokumentationsvorrichtung einschließlich der Sensoren zu ermöglichen. Weiter vorzugsweise erfolgt der Betrieb der Dokumentationsvorrichtung in Intervallen, insbesondere in Perioden von weniger als 60 s, weniger als 30 s, weniger als 10 s, oder weniger als 5 s.

Vorzugsweise umfaßt die Dokumentationsvorrichtung weiter eine Signalverarbeitungsvorrichtung, insbesondere einen Microcontroller bzw. eine CPU.

Vorzugsweise ist die Dokumentationsvorrichtung flexibel. Flexibel bedeutet in diesem Zusammenhang, daß die Dokumentationsvorrichtung biegsam, bevorzugt rückstellfähig biegsam, ist. Insbesondere umfaßt die Dokumentationsvorrichtung eine flexible Leiterplatte. Die Dokumentationsvorrichtung, insbesondere ausschließlich der Sensoren, ist vorzugsweise dünner als 5 mm, weiter vorzugsweise dünner als 3 mm, insbesondere dünner als 2 mm. Besonders bevorzugt ist die längste Kantenlänge der Dokumentationsvorrichtung, insbesondere ausschließlich der Sensoren, vorzugsweise kleiner als 10 cm, weiter vorzugsweise kleiner als 5 cm, insbesondere kleiner als 2 cm.

Weiter bevorzugt ist die Dokumentationsvorrichtung ausgelegt, zusammen mit dem Behälter sterilisierbar zu sein. Insbesondere ist die Dokumentationsvorrichtung mittels Dampf, Ethylenoxid, Ethanol und/oder gamma-Bestrahlung sterilisierbar. D.h. die Dokumentationsvorrichtung ist ausgelegt, beim Autoklavieren mit Temperaturen bis etwa 100 °C, bevorzugt bis etwa 120 °C, weiter bevorzugt bis etwa 150°C im wesentlichen formstabil und funktionsfähig zu bleiben. Besonders bevorzugt ist die Dokumentationsvorrichtung ausgelegt nicht durch Ethylalkohol lösbar zu sein, insbesondere ist die Dokumentationsvorrichtung fluiddicht. Weiter vorzugsweise ist die Dokumentationsvorrichtung ausgelegt, nach einer Sterilisation durch gamma-Strahlung funktionsfähig zu sein. Insbesondere ist die Dokumentationsvorrichtung gegenüber gamma-Strahlung abgeschirmt.

Vorzugsweise ist die Dokumentationsvorrichtung durch Verschweißen und/oder Verkleben und/oder Einbetten unlösbar mit dem Behälter verbunden. D.h. die Dokumentationsvorrichtung ist bevorzugt durch Anschweißen, Einschweißen, teilweises oder vollständiges Einbetten und/oder Kleben an bzw. in dem Behälter befestigt, insbesondere derart befestigt, daß das Behältnissystem durch das Ablösen der Dokumentationsvorrichtung unbrauchbar wird.

Ein weiterer Aspekt der vorliegenden Erfindung betrifft die Verwendung eines erfindungsgemäßen Behältnissystems als Lagerbehältnis und/oder Reaktionsbehältnis und/oder disposable container in einer dokumentationspflichtigen Anwendung.

Die Dokumentationspflicht kann sich insbesondere auf alle ursprünglichen Laboraufzeichnungen beziehen, die vollständig erfaßt, lesbar und zur Rekonstruktion einer Prüfung notwendig und geeignet sein müssen. Dazu zählen insbesondere eine eindeutige Zuordnung der erhobenen Daten zu der Person, welche die Daten erhoben hat, eine vollständige Rückverfolgbarkeit von Änderungen (Datum, Uhrzeit, Person, welche die Änderung vorgenommen hat, Gründe für Änderung), Erhalt der ursprünglichen Daten nach einer Änderung. Vorteilhafterweise werden alle diese Anforderungen durch das erfindungsgemäße Behältnissystem erfüllt.

Ein weiterer Aspekt der vorliegenden Erfindung betrifft ein Verfahren zur Herstellung eines Behältnissystems umfassend die Schritte:
- Bereitstellen eines Behälters,
- Bereitstellen einer elektronischen Dokumentationsvorrichtung mit
   -- einer Zeitgebereinrichtung zum Erfassen eines Zeitpunkts,
   -- zumindest einer Sensorvorrichtung und
   -- zumindest einer Eingabevorrichtung, die ausgelegt ist, eine manuelle Betätigung zu erfassen und
   -- einer Speichereinrichtung, welche ausgelegt ist, zumindest einen von der zumindest einen Sensorvorrichtung erfaßten Meßwert und/oder eine von der zumindest einen Eingabevorrichtung erfaßte Eingabe auslesbar zu speichern,
   wobei dem zumindest einen Meßwert und/oder der zumindest einen Eingabe ein dessen/deren Erfassung entsprechender Zeitpunkt mittels der Zeitgeber-einrichtung zugeordnet ist und
- Anordnen der Dokumentationsvorrichtung an den Behälter.

Das Anordnen der Dokumentationsvorrichtung kann bevorzugt ein Befestigen bzw. Verbinden, insbesondere ein unlösbares Befestigen bzw. Verbinden, umfassen.

Vorzugsweise umfaßt das Verfahren weiter den Schritt: zumindest bereichsweises Anbringen der zumindest einen Sensorvorrichtung an den Behälter.

Vorzugsweise umfaßt das Verfahren weiter den Schritt: Durchführen eines Zeitabgleichs der Zeitgebereinrichtung. Der Zeitabgleich kann derart durchgeführt werden, daß die Zeitgebereinrichtung mit einer externen Zeitgebereinrichtung dadurch synchronisiert wird, daß die aktuelle Zeit der externen Zeitgebereinrichtung in die Zeitgebereinrichtung der Dokumentationsvorrichtung geschrieben wird, beispielsweise mittels der Eingabevorrichtung. Alternativ kann die aktuelle Zeit der externen Zeitgebereinrichtung in der Speichereinrichtung zusammen mit der aktuellen Zeit der Zeitgebereinrichtung gespeichert werden, um die Zeitdifferenz zu dokumentieren. Bevorzugt erfolgt die Dokumentation der Zeitdifferenz periodisch, um einen Zeitgang der Zeitgebereinrichtung zu dokumentieren. Die externe Zeitquelle kann insbesondere ein per Rundfunk, z. B. DCF77, übertragenes Zeitsignal sein.

Vorzugsweise umfaßt das Verfahren weiter den Schritt: Verschließen des Behälters mittels einer Versiegelungseinrichtung. Insbesondere kann das Verschließen steril und/oder fluiddicht erfolgen.

Vorzugsweise umfaßt das Verfahren weiter den Schritt: Sterilisieren des Behältnissystems. Bevorzugt erfolgt die Sterilisation durch Autoklavieren, mittels Sterilisationsmittel (z. B. Ethylenoxid und/oder Ethylalkohol) bzw. Bestrahlen mit gamma-Strahlung.

Vorzugsweise umfaßt das Verfahren weiter den Schritt: Speichern des Herstellungsdatums und/oder der Chargennummer und/oder des Ablaufdatums in der Speichereinrichtung.

Die vorangehende Beschreibung der Aspekte der Erfindung ist nicht auf die jeweiligen Aspekte beschränkt. Vielmehr gelten die Ausführungen zu den jeweiligen Aspekten sinngemäß für die weiteren Aspekte der Erfindung. Insbesondere gelten die Ausführungen in Hinsicht auf das Behältnissystem auch für die Verwendung und umgekehrt sowie für das Verfahren bzw. bevorzugte Ausführungsformen bzw. Ausführungsvarianten hiervon.

### Figurenbeschreibung

Nachfolgend werden bevorzugte Ausführungsformen der vorliegenden Erfindung anhand begleitender Figuren beispielhaft beschrieben. Einzelne Elemente der beschriebenen Ausführungsformen sind nicht auf die jeweilige Ausführungsform beschränkt. Vielmehr können Elemente der Ausführungsformen beliebig miteinander kombiniert werden und neue Ausführungsformen dadurch erstellt werden. Es zeigt
- Figur 1:: eine Draufsicht auf ein Behältnissystem,
- Figur 2:: eine Detailansicht einer Dokumentationsvorrichtung,
- Figur 3:: ein Flußdiagramm der Zustandserfassung,
- Figur 4:: ein Flußdiagramm der Erfassung eines Versiegelungsbruchs,
- Figur 5:: ein Flußdiagramm der Erfassung eines Sensormeßwertes.

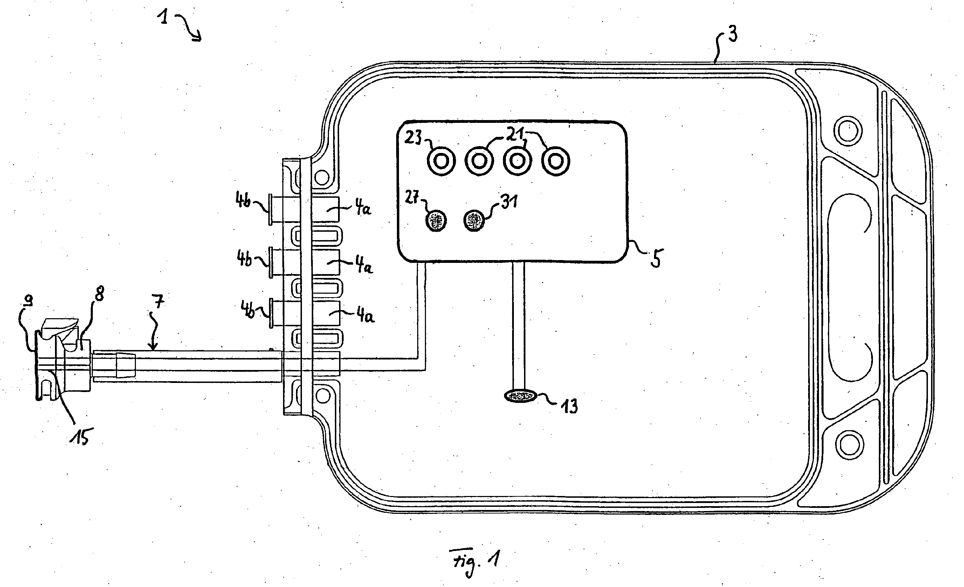

**Figur 1** zeigt eine Draufsicht auf ein Behältnissystem 1. Die gezeigte bevorzugte Ausführungsform umfaßt einen Behälter 3, der als sterilisierbarer Einwegbeutel aus flexiblem Kunststoff ausgebildet ist. Dieser bevorzugte Behälter ist ausgelegt, im medizinischen und/oder biotechnologischen Bereich eingesetzt zu werden und weist deshalb Anschlüsse 4a mit sterilen Abreißlaschen 4b auf, um den Behälter 3 zu befüllen bzw. zu entleeren.

An einem der Anschlüsse 4a ist eine Schlauchverbindung 7 angeordnet, an deren Ende ein Schlauchverbinder 8 den Abschluß bildet. Der Schlauchverbinder 8 weist eine Versiegelungseinrichtung 9 mit einer fluiddichten Metallfolie auf, welche das Innere des Schlauchverbinders 8, der Schlauchverbindung 7 und des Behälters 3 steril vom Äußeren trennt. Alternativ oder zusätzlich zu der Metallfolie kann Versiegelungseinrichtung 9 auch eine Folie aus einem elektrisch leitfähigen Polymer umfassen.

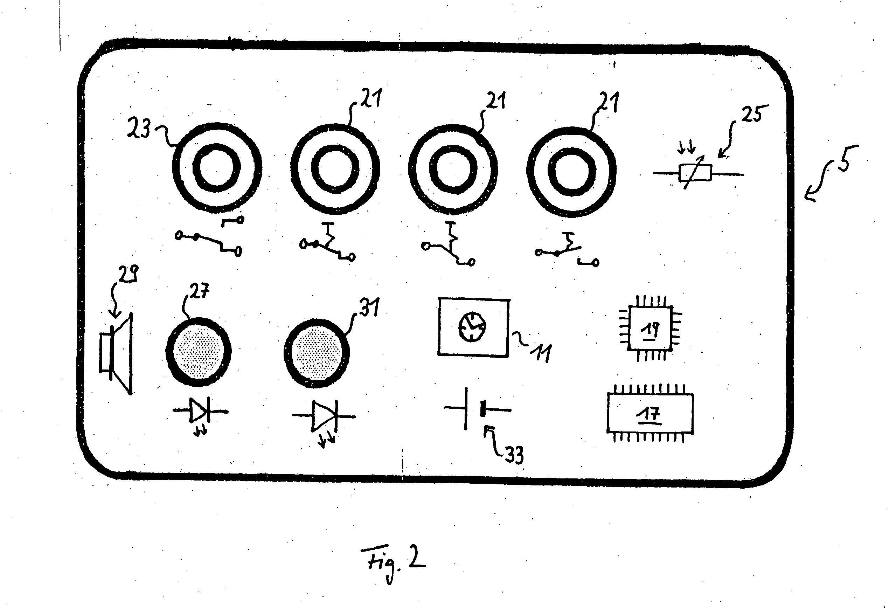

Mit dem Behälter 3 ist eine elektronische Dokumentationsvorrichtung 5 (in Figur 2 im Detail gezeigt) unlösbar verbunden. D.h. die Dokumentationsvorrichtung 5 ist bevorzugt durch Anschweißen, Einschweißen und/oder Kleben derart an dem Behälter 3 befestigt, daß das Behältnissystem 1 durch das Ablösen der Dokumentationsvorrichtung 5 sichtbar unbrauchbar wird. Insbesondere wird der Behälter 3 zerstört.

Die Dokumentationsvorrichtung 5 umfaßt in dieser Ausführungsform zwei Sensorvorrichtungen und zwar einen Temperatursensor 13 und einen elektrischen Leitfähigkeitssensor 15. Der Temperatursensor 13 ist derart an dem Behälter 3 angeordnet ist, daß der Temperatursensor 13 im wesentlichen die Temperatur des Inhalts des Behälters 3 erfaßt. Der elektrische Leitfähigkeitssensor 15 ist an dem Schlauchverbinder bzw. der Versiegelungseinrichtung 9 derart angeordnet, daß der elektrische Leitfähigkeitssensor 15 im wesentlichen die elektrischen Leitfähigkeit der Metallfolie der Versiegelungseinrichtung 9 erfaßt. Eine Änderung der elektrischen Leitfähigkeit der Metallfolie, insbesondere eine Verringerung der elektrischen Leitfähigkeit, wird durch den elektrischen Leitfähigkeitssensor 15 erfaßt, wobei dieses Ereignis als möglicher Bruch der Versiegelung durch die Dokumentationsvorrichtung 5 dokumentiert wird.

Alternativ oder zusätzlich zu dem Temperatursensor 13 und dem elektrischen Leitfähigkeitssensor 15 kann die Dokumentationsvorrichtung 5 ebenfalls einen Photosensor und/oder einen elektrischen Widerstandssensor und/oder einen pH-Sensor und/oder einen Sensor zur Bestimmung der Konzentration gelöster Gase und/oder der Konzentration von im Behälterinneren vorliegender Substanzen (beispielsweise Sauerstoff, Kohlendioxid und/oder Chlorophyll) und/oder einen Sensor zur Bestimmung einer Partikel- und/oder Zelldichte umfassen. Insbesondere kann der Photosensor ausgelegt sein, eine photometrische Messung durchzuführen. Dazu kann der Photosensor in einem definierten Abstand von einer Lichtquelle angeordnet sein, beispielsweise um eine Trübung oder einen Farbumschlag aufgrund einer chemischen Reaktion mittels der auftretenden photometrischen Extinktion zu bestimmen. Bevorzugt umfaßt die zumindest eine Sensorvorrichtung einen nicht-invasiven Sensor, d.h. einen Sensor, welcher die Messung eines Parameters erlaubt, ohne die Integrität des Behälters aufzuheben bzw. ohne das Innere des Behälters zu kontaktieren bzw. in dieses zumindest teilweise einzudringen. Bevorzugt ist ein nicht-invasiver Sensor an bzw. auf bzw. in Nähe der äußeren Wandung des Behälters 3 angeordnet bzw. anordenbar. Insbesondere sind die nicht-invasiven Sensoren als Einwegsensoren ausgebildet.

**Figur 2** zeigt eine Detailansicht einer Dokumentationsvorrichtung 5. Die gezeigte Dokumentationsvorrichtung 5 umfaßt drei Taster 21, einen Schalter 23, sowie eine Empfangsvorrichtung 25 als bevorzugte Eingabevorrichtungen 21, 23, 25. Desweiteren umfaßt die Dokumentationsvorrichtung 5 eine Zeitgebereinrichtung 11 und eine Speichereinrichtung 17, welche ausgelegt ist, die von dem Temperatursensor 13 und von dem elektrischen Leitfähigkeitssensor 15 erfaßten Meßwerte sowie die von den Eingabevorrichtungen 21, 23, 25 erfaßten Eingaben zusammen mit der von der Zeitgebereinrichtung 11 bereitgestellten Erfassungszeit auslesbar zu speichern.

Die Taster 21 und der Schalter 23 sind ausgelegt, um eine manuelle Betätigung durch einen Benutzer zu erfassen. Eine manuelle Betätigung umfaßt insbesondere das im wesentlichen kraftlose Berühren der Taster 21. Dazu können die Taster 21 beispielsweise als Photowiderstand 21 ausgebildet sein, welcher eine Abdeckung - d.h. eine kraftlose Berührung - durch einen Finger detektieren kann. Weiter können die Taster 21 als elektrische Kontaktsensor 21 ausgebildet sein, der eine elektrische Überbrückung durch die Haut eines Fingers detektieren kann. Dagegen kann der Schalter 23 beispielsweise als Schiebeschalter 23 ausgebildet sein, welcher seine Stellung durch das Aufbringen einer Kraft durch einen Benutzer ändert. Mittels der Taster 21 können z. B. auf einfache Weise Bestätigungen durch die Dokumentationsvorrichtung 5 erfaßt werden.

Die Empfangsvorrichtung 25 ist ausgelegt, um ein Signal einer externen Vorrichtung zu erfassen. In dieser Ausführungsform umfaßt die Empfangsvorrichtung einen Infrarotsensor, um mit einer externen Vorrichtung mittels einer Infrarot-Verbindung zu kommunizieren. Insbesondere können numerische bzw. alpha-numerische Benutzereingaben mittels einer externen Tastatur und/oder eines externen Computers erfaßt, an die Empfangsvorrichtung übertragen und durch die Dokumentationsvorrichtung gespeichert werden. Alternativ oder zusätzlich kann die Empfangsvorrichtung eine Antenne, eine Spule, einen elektrischen Verbinder, einen Glasfaserverbinder etc. umfassen.

Die gezeigte Dokumentationsvorrichtung 5 umfaßt eine optische Anzeige 27, eine akustische Anzeige 29 als bevorzugte Anzeigevorrichtung sowie eine Sendevorrichtung 31. Die optische Anzeige umfaßt eine Leuchtdiode 27. Alternativ oder zusätzlich kann die optische Anzeige ebenfalls eine alpha-numerische Anzeige, wie beispielsweise eine 7-Segment-Anzeige oder ein LCD-Display umfassen. Die akustische Anzeige 29 umfaßt bevorzugt eine piezoelektrische Schallquelle 29.

Die Sendevorrichtung 31 ist ausgelegt, um einen Zustand der Dokumentationsvorrichtung 5 und/oder einen in der Speichereinrichtung 17 gespeicherten Inhalt an eine externe Vorrichtung, beispielsweise einen Computer, zu senden. Als eine bevorzugte Ausführungsform kann die Sendevorrichtung 31 eine Infrarot-Diode umfassen, um mit der externen Vorrichtung mittels einer Infrarot-Verbindung zu kommunizieren. Alternativ oder zusätzlich kann die Sendevorrichtung 31 auch eine Antenne, eine Spule, einen elektrischen Verbinder, einen Glasfaserverbinder etc. umfassen.

Insbesondere kann eine Sendevorrichtung 31 identisch zu einer Empfangsvorrichtung 25 sein. Insbesondere kann eine Antenne oder ein elektrischer Verbinder sowohl eine Sendevorrichtung 31 als auch eine Empfangsvorrichtung 25 im Sinne der Erfindung sein.

Die Speichereinrichtung 17 ist dazu ausgelegt Daten, wie die Erfassungszeit, einen Meßwert und/oder eine Eingabe, auslesbar zu speichern. Insbesondere kann die Speichereinrichtung 17 ein EPROM, ein EEPROM oder einen Flash-Speicher umfassen, wobei der (Schreib-/Lese-)Zugriff auf die Speichereinrichtung 17 mittels einer Signalverarbeitungsvorrichtung 19, beispielsweise einem Microcontroller, erfolgen kann.

Um die Dokumentationsvorrichtung 5 mit Energie zu versorgen, umfaßt die Dokumentationsvorrichtung 5 eine Stromquelle 33. Die Stromquelle 33 umfaßt insbesondere eine Batterie 33 bzw. einen Akkumulator 33, welche(r) ausgelegt ist, die Dokumentationsvorrichtung 5 zumindest bis zum Verfallsdatum des Behältnissystems 1 mit Strom zu versorgen.

Alternativ oder zusätzlich kann die Stromquelle 33 eine Stromerzeugereinrichtung umfassen, die kontinuierlich oder zu diskreten Zeiten Strom erzeugt, der insbesondere in einem Akkumulator und/oder Kondensator gespeichert werden kann. Eine Stromerzeugereinrichtung kann beispielsweise eine Solarzelle und/oder ein induktives Bauteil (z. B. eine Spule) sein, wobei eine Solarzelle auftreffendes Licht und das induktive Bauteil ein außen angelegtes magnetisches Wechselfeld in einen elektrischen Strom umwandelt.

Ein beispielhafter Lebenslauf des Behältnissystems 1 in einer bevorzugten Ausführungsform sei im folgenden beschrieben, wobei Merkmale, die identisch zu den mit Bezug auf die Figuren 1 und 2 beschriebenen Merkmalen sind, mit den dort verwendeten Bezugszeichen versehen sind. Die im folgenden beschriebenen und durch die Dokumentationsvorrichtung 5 zumindest teilweise gespeicherten Ereignisse sind in der nachfolgenden Tabelle 1 als beispielhaftes Produkt- bzw. Chargenprotokoll bzw. "Batch Record" zusammengefaßt.

Ein Behälter 3 wird während des Herstellungsprozesses mit der Dokumentationsvorrichtung 5 unlösbar verbunden. Der Behälter 3 weist dabei eine Schlauchverbindung 7 mit einem Schlauchverbinder 8 auf, wobei der Schlauchverbinder 8 eine Versiegelungseinrichtung 9 aufweist. Ein Temperatursensor 13 wird mit dem Behälter 3 und ein elektrischer Leitfähigkeitssensor 15 mit der Versiegelungseinrichtung 9 verbunden.

Die Dokumentationseinrichtung 5 weist eine RFID-Antenne als bevorzugte Sende- und Empfangsvorrichtung auf, über die von einer externen Einrichtung die Seriennummer, das Herstellungsdatum und das Verfallsdatum des Behältnissystems 1 in eine Speichereinrichtung 17 gespeichert wird. Eine Zeitgebereinrichtung wird mittels einer externen Zeitquelle synchronisiert. Es versteht sich, daß anstatt der RFID-Antenne auch andere Sende- und Empfangsvorrichtungen verwendet werden können, beispielsweise Sende- und Empfangsvorrichtungen, die geeignet sind, eine Infrarot-, Bluetooth-, Ethernet, Firewire- und/oder USB-Verbindung zu einer externen Vorrichtung herzustellen.

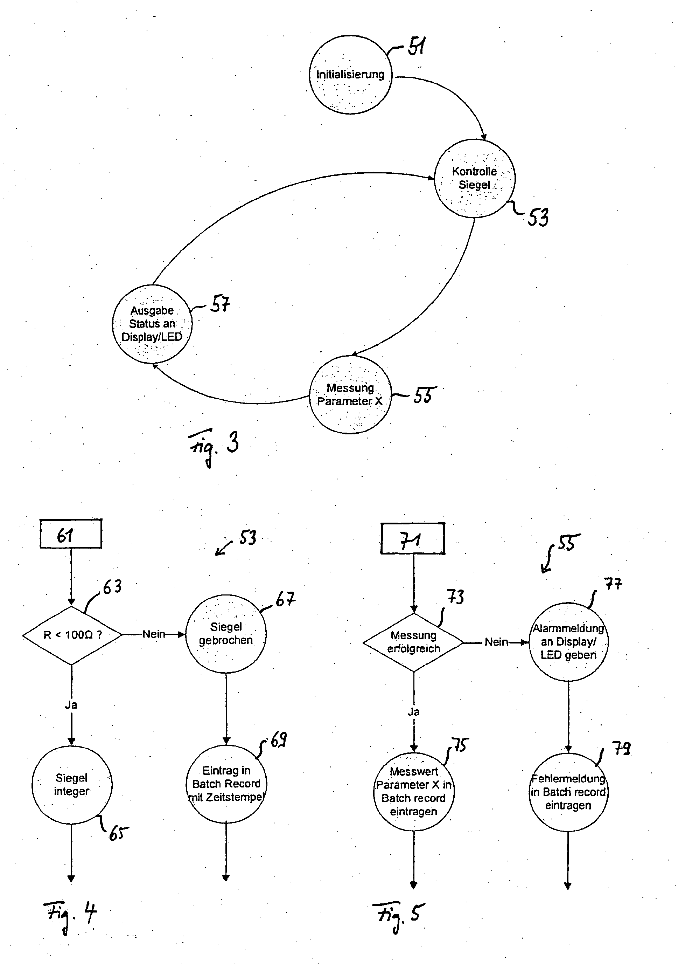

Das Behältnissystem 1 wird verpackt und sterilisiert. Wie in **Figur 3** gezeigt, werden durch den Hersteller die Produktdaten, insbesondere das Datum der Herstellung und das Verfalls- bzw. Haltbarkeitsdatum, als Anfangsereignis bzw. erstes Ereignis bzw. als Initialisierungsschritt 51 in dem Produktprotoköll (siehe Tabelle 1, Zeile 1) dokumentiert, wobei bevorzugt das Behältnissystem zunächst mit einem Sperrvermerk versehen wird (in Tabelle 1: Status "G" für "Gesperrt"). Nach der Herstellung wird das Behältnissystem in der Qualitätskontrolle geprüft und nach erfolgreicher Prüfung gelagert. Das Ergebnis der Qualitätskontrolle wird in einem Freigabeprötokoll an die Dokumentationsvorrichtung 5, insbesondere als PDF-Dokument über die RFID-Antenne, übertragen und dort zumindest teilweise gespeichert (siehe Tabelle 1, Zeile 2). Ein durch den Hersteller freigegebenes Behältnissystem erhält den Status "OK" und kann anschließend an einen Benutzer ausgeliefert werden.

Die Dokumentationsvorrichtung 5 führt nach der Initialisierung kontinuierlich bzw. periodisch einen Versiegelungszustandserfassungsschritt 53 und/oder einen Meßwerterfassungsschritt 55 durch, wobei in einem Statusanzeigeschritt 57 der Status des Behältnissystems 1 mittels einer Anzeigevorrichtung bevorzugt angezeigt wird. Dabei kann insbesondere das grüne Leuchten einer LED 27 anzeigen, daß der Status des Behältnissystems auf "OK" gesetzt ist.

Der Benutzer kann über eine drahtlose Verbindung mittels der RFID-Antenne der Dokumentationsvorrichtung 5 eine Bestandsaufnahme durchführen oder eine Statusinformation (Empfangen am ... / Lagerort / etc.) in der Speichereinrichtung speichern (siehe Tabelle 1, Zeile 3).

Zur Verwendung wird das Behältnissystem 1 entpackt und auf die Verwendbarkeit geprüft. Ein erfolgreiche Prüfung kann in der Dokumentationseinrichtung 5 protokolliert werden. Ein Taster 21 erlaubt die Speicherung einer Verwendungsfreigabe inklusive der Freigabezeit durch einen Benutzer nach einer erfolgten und erfolgreichen visuellen Inspektion des Behältnissystems 1 (siehe Tabelle 1, Zeile 4). Die erfolgreiche Speicherung dieses Ereignisses erfolgt über ein grünes Aufleuchten einer LED 27. Bei fehlerhafter Funktion erfolgt ein rotes Aufleuchten der LED 27 und/oder eine akustische Rückmeldung über einen Piezo-Lautsprecher 29.

Für einen Prozeßschritt muß das Behältnissystem 1 manuell vorbereitet werden, z. B. durch ein Befüllen des Behälters 3 mit einer Probe. Das Befüllen erfordert eine Entsiegelung der Versiegelungseinrichtung 9. Die Entsiegelung wird während des in **Figur 4** dargestellten Versiegelungszustandserfassungsschritts 53 detektiert bzw. erfaßt, wobei dieses Ereignis zusammen mit der Erfassungszeit, d.h. dem Zeitpunkt der Entsiegelung, in der Speichereinrichtung 17 gespeichert wird (siehe Tabelle 1, Zeile 5).

Der Versiegelungszustandserfassungsschritt 53 umfaßt vorzugsweise die kontinuierliche bzw. periodische Messung der elektrischen Leitfähigkeit bzw. des elektrischen Widerstandes R der Versiegelungseinrichtung 9 bzw. eines elektrischen Leiters davon mittels einer Sensorvorrichtung 15 bevorzugt in einem Widerstandserfassungsschritt 61. Unterschreitet der elektrische Widerstand bei einem Vergleichsschritt 63 einen speziellen (vorbestimmten bzw. vorbestimmbaren) Schwellwert, beispielsweise 100 Ohm, so gibt die Sensorvorrichtung 15 in einem Ausgabeschritt 65 die Zustandsmeldung "Siegel integer" aus. Überschreitet der elektrische Widerstand den speziellen (vorbestimmten bzw. vorbestimmbaren) Schwellwert, so gibt die Sensorvorrichtung 15 in einem Ausgabeschritt 67 die Zustandsmeldung "Siegel gebrochen" aus. Die Dokumentationsvorrichtung protokolliert den Bruch der Versiegelung zusammen mit der Erfassungszeit in Schritt 69.

Vorzugsweise wird nach der Entsiegelung eine Bestätigung durch den Benutzer erforderlich, um eine gebrauchsnotwendige Entsiegelung zu bestätigen und diese von einer versehentlichen Entsiegelung zu differenzieren. Dies kann vorzugsweise derart erfolgen, daß die Dokumentationsvorrichtung automatisch den Status des Behältnissystems 1 von "OK" auf "Gesperrt" setzt, wenn die Entsiegelung detektiert und protokolliert wird (siehe Tabelle 1, Zeile 5). Ein akustisches und/oder optisches Signal fordert den Benutzer auf, die Entsiegelung zu bestätigen. Erfolgt die Bestätigung durch den Benutzer innerhalb einer vorgegebenen Zeit, vorzugsweise innerhalb von weniger als einer Minute oder von weniger als 30 Sekunden, insbesondere mittels eines Tasters 21, so wird die Bestätigung in der Dokumentationsvorrichtung 5 protokolliert und der Status des Behältnissystems 1 auf "OK" gesetzt (siehe Tabelle 1, Zeile 6).

Das Behältnissystem kann nun durch einen Benutzer mit einem Medium zumindest teilweise befüllt werden, wobei Angaben über die Befüllungszeit, die Art und/oder die Menge des Mediums in der Dokumentationseinrichtung gespeichert werden (siehe Tabelle 1, Zeile 7). Die Temperatur der Probe in dem Behälter 3 wird kontinuierlich bzw. periodisch, insbesondere in zeitlichen Perioden von etwa 10 s, durch den Temperatursensor 13 gemessen und der erfaßte Meßwert zusammen mit der Erfassungszeit in der Speichereinrichtung 17 gespeichert. Über eine drahtlose Verbindung können die erfaßten Meßwerte mittels der RFID-Antenne zu einer externen Vorrichtung zwecks weiterer Verarbeitung übertragen werden.

**Figur 5** zeigt die einzelnen Schritte der Meßwerterfassung 55, die beispielsweise die kontinuierliche bzw. periodischen Messung der Temperatur mittels des Temperatursensors 13 in einem Sensormeßwerterfassungsschritt 71 umfaßt. Ob die Erfassung des Sensormeßwertes erfolgreich durchgeführt wurde, wird in einem Vergleichsschritt 73 ermittelt. Bleibt beispielsweise der gemessene Widerstand eines NTC- oder PTC-Temperaturfühlers bzw. die daraus berechnete Temperatur innerhalb vorbestimmter Grenzen, d.h. oberhalb und/oder unterhalb spezieller (vorgegebener bzw. vorgebbarer) Werte, so gibt die Sensorvorrichtung 13 in einem Ausgabeschritt 75 den entsprechenden Meßwert aus, welcher durch die Dokumentationsvorrichtung 5 gespeichert bzw. dokumentiert wird. Ist die Messung jedoch nicht erfolgreich, z. B. da der Temperatursensor offen oder kurzgeschlossen ist, gibt die Sensorvorrichtung 13 in einem Ausgabeschritt 77 eine Fehlermeldung aus, welche durch die Dokumentationsvorrichtung zusammen mit der Erfassungszeit in Schritt 79 protokolliert bzw. dokumentiert wird.

Nach dem Entleeren des Behälters 3 oder dem Abschluß des Prozesses durch den Benutzer (siehe Tabelle 1, Zeile 8) kann das Behältnissystem 1 für die weitere Verwendung, insbesondere wenn der Inhalt des Behältnissystems 1 gelagert werden soll, beispielsweise durch Betätigen eines Schalters 23 gesperrt werden (siehe Tabelle 1, Zeile 9), d.h. der Status des Behältnissystems wird von "OK" auf "G" für "gesperrt" gesetzt. Die entsprechende Statusänderung (Behälter gesperrt am ...") wird in der Speichereinrichtung 17 zusammen mit der Erfassungszeit gespeichert. Eine unbeabsichtigte Weiterverwendung kann so vorteilhafterweise verhindert werden.

Soll der Inhalt des Behältnissystems 1 weiter verwendet werden, so kann beispielsweise eine Freigabe zu Produktionszwecken erfolgen, welche durch die Dokumentationsvorrichtung 5 protokolliert bzw. dokumentiert wird (siehe Tabelle 1, Zeile 10). Erreicht das Behältnissystem 1 oder der darin enthaltene Inhalt die in der Dokumentationsvorrichtung 5 gespeicherte Haltbarkeitsdauer bzw. das Verfallsdatum, so generiert die Dokumentationsvorrichtung 5, insbesondere automatisch bzw. ohne Benutzereingriff eine Systemmeldung, die protokolliert wird, wobei der Status des Behältnissystems 1 auf "Gesperrt" gesetzt wird. Eine akustische und/oder optische Anzeige 27, 29 kann die Sperrung des Behältnissystems 1 anzeigen (siehe Tabelle 1, Zeile 11).

Vor der endgültigen Entsorgung des Behältnissystems 1 wird die in der Speichereinrichtung 17 gespeicherte Historie des Behältnissystems ausgelesen, insbesondere drahtlos mittels der RFID-Antenne, und auf einem externen Datenträger gespeichert (siehe Tabelle 1, Zeile 12).

**Tabelle 1:**

| Nr. | Zeitstempel | Aktion | Urheber | Status | Eintrag | Zugriff |
|---|---|---|---|---|---|---|
| 1 | 04.03.2008 | Initialisierung | Hersteller | G | Produktdaten gespeichert | via Empfangsvorrichtung |
| | 10:25:30 | | | | | |
| 2 | 04.03.2008 | Freigabe des Behältnissystems zur Auslieferung | Hersteller | OK | Freigabeprotokoll gespeichert | via Empfangsvorrichtung |
| | 15:10:12 | | | | | |
| 3 | 06.04.2008 | Lieferung | Benutzer Y | OK | Lieferungsempfang gespeichert | via Eingabevorrichtung |
| | 00:25:21 | | | | | |
| 4 | 06.04.2008 | Freigabe des Behältnissystems für die Verwendung | Benutzer X | OK | Verwendungsfreigabe gespeichert | via Eingabevorrichtung |
| | 09:20:30 | | | | | |
| 5 | 07.04.2008 | Entsiegelung | Produkt | G | Entsiegelung gespeichert | automatisch erzeugte Systemmeldung, Anzeige |
| | 06:10:45 | | | | | |
| 6 | 07.04.2008 | Entsiegelung bestätigt | Benutzer Z | OK | Verwendungsfreigabe gespeichert | via Eingabevorrichtung |
| | 06:11:00 | | | | | |
| 7 | 07.04.2008 | Befüllung | Benutzer Z | OK | Befüllen und Art des Inhalts gespeichert | via Eingabevorrichtung |
| | 07:15:25 | | | | | |
| 8 | 08.04.2008 | Abschluß einer manuellen Operation | Benutzer Z | OK | Medien- und Prozeßinformationen | via Eingabevorrichtung |
| | 09:10:23 | | | | | |
| 9 | 08.04.2008 | Transfer in Lager | Benutzer Y | G | Transferdatum und Haltbarkeitsdauer | via Eingabevorrichtung |
| | 12:10:04 | | | | | |
| 10 | 12.05.2008 | Freigabe zu Produktionszwecken | Benutzer Y | OK | Freigabegrund und -zweck gespeichert | via Eingabevorrichtung, via Empfangsvorrichtung |
| | 15:10:01 | | | | | |
| 11 | 08.06.2008 | Erreichen der maximalen Lagerdauer | Produkt | G | Verwendung gesperrt wegen Erreichen des Haltbarkeitsdatums | automatisch erzeugte Systemmeldung, Anzeige, Meldung via Sendevorrichtung |
| | 12:10:04 | | | | | |
| 12 | 10.06.2008 | Freigabe zur Entsorgung | Benutzer W | OK | "Batch Record" wird archiviert | via Sendevorrichtung |
| | 08:00:11 | | | | | |

### Bezugszeichenliste

- 1: Behältnissystem
- 3: Behälter
- 4a: Anschluß
- 4b: Abreißlasche
- 5: Dokumentationsvorrichtung
- 7: Schlauchverbindung
- 8: Schlauchverbinder
- 9: Versiegelungseinrichtung
- 11: Zeitgebereinrichtung
- 13: Temperatursensor
- 15: Widerstandssensor
- 17: Speichereinrichtung
- 19: Signalverarbeitungsvorrichtung
- 21: Taster
- 23: Schalter
- 25: Empfangsvorrichtung
- 27: optische Anzeige
- 29: akustische Anzeige
- 31: Sendevorrichtung
- 33: Stromquelle

## Patentansprüche

1. Behältnissystem (1), insbesondere Aufbewahrungs- und/oder Reaktionsbehältnissystem für Flüssigkeiten, umfassend:
- einen Behälter (3) und
- eine mit dem Behälter (3) befestigte elektronische Dokumentationsvorrichtung (5), wobei die Dokumentationsvorrichtung (5) weiter umfaßt:
-- eine Zeitgebereinrichtung (11) zum Erfassen eines Zeitpunkts,
-- zumindest eine Sensorvorrichtung (13; 15)
-- zumindest eine Eingabevorrichtung (21; 23; 25), die ausgelegt ist, eine manuelle Betätigung zu erfassen,
-- eine Speichereinrichtung (17), welche ausgelegt ist, zumindest einen von der zumindest einen Sensorvorrichtung (13; 15) erfaßten Meßwert und/oder zumindest eine von der zumindest einen Eingabevorrichtung (21; 23; 25) erfaßte Eingabe auslesbar zu speichern und
-- zumindest eine Anzeigevorrichtung (27; 29), um einem Benutzer einen Zustand der Dokumentationsvorrichtung (5) und/oder einen in - der Speichereinrichtung (17) gespeicherten Inhalt anzuzeigen,
wobei dem zumindest einen Meßwert und/oder der zumindest einen Eingabe ein deren Erfassung entsprechender Zeitpunkt mittels der Zeitgebereinrichtung (11) zugeordnet ist.

2. Behältnissystem (1) nach Anspruch 1, wobei der Behälter (3) aus einem flexiblen Kunststoff besteht.

3. Behältnissystem (1) nach Anspruch 1 oder 2, wobei der Behälter (3) ein Einwegbehälter (3), bevorzugt ein sterilisierbarer Einwegbehälter (3), ist.

4. Behältnissystem (1) nach einem der vorigen Ansprüche, wobei die zumindest eine Eingabevorrichtung (21; 23; 25), die ausgelegt ist, eine manuelle Betätigung zu erfassen, zumindest einen Taster (21) und/oder zumindest einen Schalter (23) umfaßt.

5. Behältnissystem (1) nach einem der vorherigen Ansprüche, wobei die zumindest eine Eingabevorrichtung (21; 23; 25) eine Vielzahl von Eingabevorrichtungen umfasst,
wobei die Eingabevorrichtungen (21, 23, 25) eine Empfangsvorrichtung (25) umfassen, die ausgelegt ist, ein Signal einer externen Vorrichtung zu erfassen,
wobei die Empfangsvorrichtung (25) ein elektromagnetisches Kopplungselement und/oder ein kapazitives Kopplungselement und/oder ein induktives Kopplungselement und/oder ein galvanisches Kopplungselement umfassen kann.

6. Behältnissystem (1) nach einem der vorigen Ansprüche, wobei der Behälter (3) eine Versiegelungseinrichtung (9) aufweist, wobei die Versiegelungseinrichtung (9) eine Folie und/oder einen Leiter aus Metall und/oder ein elektrisch leitfähiges Polymer umfassen kann.

7. Behältnissystem (1) nach Anspruch 6, wobei die zumindest eine Sensorvorrichtung (15) den Versiegelungszustand der Versiegelungseinrichtung (9) detektiert, wobei der zugeordnete Zeitpunkt der Erfassung einem Bruch der Versiegelungseinrichtung (9) entspricht.

8. Behältnissystem (1) nach einem der vorherigen Ansprüche, wobei die zumindest eine Sensorvorrichtung (13; 15) einen Temperatursensor (13) und/oder einen Photosensor und/oder einen elektrischen Widerstandssensor (15) und/oder einen elektrischen Leitfähigkeitssensor und/oder einen pH-Sensor umfaßt.

9. Behältnissystem (1) nach einem der vorherigen Ansprüche, wobei die Dokumentationsvorrichtung (5) weiter umfaßt:
-- zumindest eine Sendevorrichtung (31), die ausgelegt ist, um einen Zustand der Dokumentationsvorrichtung (5) und/oder einen in der Speichereinrichtung (17) gespeicherten Inhalt an eine externe Vorrichtung zu senden,
wobei die zumindest eine Sendevorrichtung (31) ein elektromagnetisches Kopplungselement und/oder ein kapazitives Kopplungselement und/oder ein induktives Kopplungselement und/oder ein galvanisches Kopplungselement umfassen kann.

10. Behältnissystem (1) nach einem der vorherigen Ansprüche, wobei die Dokumentationsvorrichtung (5) flexibel ist.

11. Behältnissystem (1) nach einem der vorherigen Ansprüche, wobei die Dokumentationsvorrichtung (5) unlösbar mit dem Behälter (3) verbunden ist, bevorzugt durch Verschweißen und/oder Verkleben und/oder Einbetten.

12. Verwendung eines Behältnissystems (1) gemäß einem der vorherigen Ansprüche als Lagerbehältnis und/oder Reaktionsbehältnis in einer dokumentationspflichtigen Anwendung.

13. Verfahren zur Herstellung eines Behältnissystems (1) umfassend die Schritte:
- Bereitstellen eines Behälters (3),
- Bereitstellen einer elektronischen Dokumentationsvorrichtung (5) mit
-- einer Zeitgebereinrichtung (11) zum Erfassen eines Zeitpunkts,
-- zumindest einer Sensorvorrichtung (13; 15),
-- zumindest einer Eingabevorrichtung (21; 23; 25), die ausgelegt ist, eine manuelle Betätigung zu erfassen,
-- einer Speichereinrichtung (17), welche ausgelegt ist, zumindest einen von der zumindest einen Sensorvorrichtung (13; 15) erfaßten Meßwert und/oder zumindest eine von der zumindest einen Eingabevorrichtung (21; 23; 25) erfaßte Eingabe auslesbar zu speichern, und
-- zumindest einer Anzeigevorrichtung (27; 29), um einem Benutzer einen Zustand der Dokumentationsvorrichtung (5) und/oder einen in der Speichereinrichtung gespeicherten Inhalt anzuzeigen,
wobei dem zumindest einen Meßwert und/oder der zumindest einen Eingabe ein deren Erfassung entsprechender Zeitpunkt mittels der Zeitgebereinrichtung (11) zugeordnet ist,
- Befestigen der Dokumentationsvorrichtung (5) an dem Behälter (3).

14. Verfahren nach Anspruch 13, weiter umfassend den Schritt:
- Durchführen eines Zeitabgleichs der Zeitgebereinrichtung (11) und/oder
- Speichern des Herstellungsdatums und/oder der Chargennummer und/oder des Ablaufdatums in der Speichereinrichtung (17).

15. Verfahren nach einem der Ansprüche 13 bis 14, weiter umfassend den Schritt:
- Verschließen des Behälters (3) mittels einer Versiegelungseinrichtung (9).

## Claims

1. A container system (1), in particular a storage and/or reaction container system for liquids, comprising:
- a container (3), and
- an electronic documentation device (5) fixed with the container (3), the documentation device (5) further comprising:
- a timer device (11) for detecting a point in time,
- at least one sensor device (13; 15)
- at least one input device (21; 23; 25) formed for detecting a manual operation,
- a storage device (17) formed for readably storing at least one measured value detected by the at least one sensor device (13; 15) and/or at least one input detected by the at least one input device (21; 23; 25), and
- at least one display device (27; 29) for displaying to a user a state of the documentation device (5) and/or a content stored in the storage device (17),
wherein a time corresponding to the time of detection of the at least one measured value and/or the at least one input is assigned to the at least one measured value and/or the at least one input by means of the timer device (11).

2. The container system (1) according to claim 1, wherein the container (3) is made of a flexible synthetic material.

3. The container system (1) according to claim 1 or 2, wherein the container (3) is a disposable container (3), preferably a sterilisable disposable container (3).

4. The container system (1) according to any one of the preceding claims, wherein the at least one input device (21; 23; 25) which is formed for detecting a manual operation comprises at least one key button (21) and/or at least on switch (23).

5. The container system (1) according to any one of the preceding claims, wherein the at least one input device (21; 23; 25) comprises a plurality of input devices,
wherein the input devices (21; 23; 25) comprise a receiving device (25) formed for receiving a signal from an external device,
wherein the receiving the device (25) can comprise an electromagnetic coupling element and/or a capacitive coupling element and/or an inductive coupling element and/or a galvanic coupling element.

6. The container system (1) according to any one of the preceding claims, wherein the container (3) has a sealing device, wherein the sealing device (9) can comprise a film and/or a conductor made of metal and/or an electrically conductive polymer.

7. The container system (1) according to claim 6, wherein the at least one sensor device (15) detects the state of sealing of the sealing device (9), with the assigned detection time corresponding to a break of the sealing device (9).

8. The container system (1) according to any one of the preceding claims, wherein the at least one sensor device (13; 15) comprises a temperature sensor (13) and/or a photosensor and/or an electrical resistance sensor (15) and/or an electrical conductivity sensor and/or a pH sensor.

9. The container system (1) according to any one of the preceding claims, the documentation device (5) further comprising:
- at least one transmitting device (31) formed for trasnmitting a state of the documentation device (5) and/or a content stored in the storage device (17) to an external device,
wherein the at least one transmitting device (31) can comprise an electromagnetic coupling element and/or a capacitive coupling element and/or an inductive coupling element and/or a galvanic coupling element.

10. The container system (1) according to any one of the preceding claims, wherein the documentation device (5) is flexible.

11. The container system (1) according to any one of the preceding claims, wherein the documentation device (5) is permanently attached to the container (3), preferably by welding and/or gluing and/or embedding.

12. The use of a container system (1) according to any one of the preceding claims as storage container and/or reaction container in an application where documentation is mandatory.

13. A method of producing a container system (1) comprising the steps of:
- providing a container (3),
- providing an electronic documentation device (5) including
- a timer device (11) for detecting a point in time,
- at least one sensor device (13; 15)
- at least one input device (21; 23; 25) formed for detecting a manual operation,
- a storage device (17) formed for readably storing at least one measured value detected by the at least one sensor device (13; 15) and/or at least one input detected by the at least one input device (21; 23; 25), and
- at least one display device (27; 29) for displaying to a user a state of the documentation device (5) and/or a content stored in the storage device (17),
- attaching the documentation device (5) at the container (3)
wherein a time corresponding to the time of detection of the at least one measured value and/or the at least one input is assigned to the at least one measured value and/or the at least one input by means of the timer device (11),
- attaching the documentation device (5) to the container (3).

14. The method according to claim 13, further comprising the step of:
- performing a time calibration of the timer device (11) and/or
- storing the production date and/or the lot number and/or the expiry date in the storage device (17).

15. The method according to one of claims 13 to 14, further comprising the step of:
- closing the container (3) by means of a sealing device (9).

## Revendications

1. Système de récipient (1), notamment système de récipient de conservation et/ou de réaction pour liquides, comprenant :
- un récipient (3) et
- un dispositif de documentation électronique (5) fixé au récipient (3), dans lequel le dispositif de documentation (5) comprend en outre :
-- un dispositif temporisateur (11) pour détecter un instant,
-- au moins un dispositif de capteur (13 ; 15),
-- au moins un dispositif de saisie (21 ; 23 ; 25) qui est conçu pour détecter un actionnement manuel,
-- un dispositif de mémoire (17) qui est conçu pour mémoriser de manière lisible au moins une valeur de mesure détectée par l'au moins un dispositif de capteur (13 ; 15) et/ou au moins une saisie détectée par l'au moins un dispositif de saisie (21 ; 23 ; 25) et
-- au moins un dispositif d'affichage (27 ; 29) pour afficher à un utilisateur un état du dispositif de documentation (5) et/ou un contenu mémorisé dans le dispositif de mémoire (17),
dans lequel un instant correspondant à leur détection est associé à l'au moins une valeur de mesure et/ou l'au moins une saisie au moyen du dispositif temporisateur (11).

2. Système de récipient (1) selon la revendication 1, dans lequel le récipient (3) se compose d'un plastique flexible.

3. Système de récipient (1) selon la revendication 1 ou 2, dans lequel le récipient (3) est un récipient jetable (3), de préférence un récipient jetable stérilisable (3).

4. Système de récipient (1) selon une des revendications précédentes, dans lequel l'au moins un dispositif de saisie (21 ; 23 ; 25) qui est conçu pour détecter un actionnement manuel comprend au moins un bouton-poussoir (21) et/ou au moins un commutateur (23).

5. Système de récipient (1) selon une des revendications précédentes, dans lequel l'au moins un dispositif de saisie (21 ; 23 ; 25) comprend une pluralité de dispositifs de saisie,
dans lequel les dispositifs de saisie (21, 23, 25) comprennent un dispositif de réception (25) qui est conçu pour détecter un signal d'un dispositif externe,
dans lequel le dispositif de réception (25) peut comprendre un élément de couplage électromagnétique et/ou un élément de couplage capacitif et/ou un élément de couplage inductif et/ou un élément de couplage galvanique.

6. Système de récipient (1) selon une des revendications précédentes, dans lequel le récipient (3) présente un dispositif de scellement (9), dans lequel le dispositif de scellement (9) peut comprendre un film et/ou un conducteur en métal et/ou un polymère électriquement conducteur.

7. Système de récipient (1) selon la revendication 6, dans lequel l'au moins un dispositif de capteur (15) détecte l'état de scellement du dispositif de scellement (9), dans lequel l'instant associé de la détection correspond à une rupture du dispositif de scellement (9).

8. Système de récipient (1) selon une des revendications précédentes, dans lequel l'au moins un dispositif de capteur (13 ; 15) comprend un capteur de température (13) et/ou un capteur photosensible et/ou un capteur de résistance électrique (15) et/ou un capteur de conductivité électrique et/ou un capteur de pH.

9. Système de récipient (1) selon une des revendications précédentes, dans lequel le dispositif de documentation (5) comprend en outre :
-- au moins un dispositif d'envoi (31) qui est conçu pour envoyer un état du dispositif de documentation (5) et/ou un contenu mémorisé dans le dispositif de mémoire (17) à un dispositif externe,
dans lequel l'au moins un dispositif d'envoi (31) peut comprendre un élément de couplage électromagnétique et/ou un élément de couplage capacitif et/ou un élément de couplage inductif et/ou un élément de couplage galvanique.

10. Système de récipient (1) selon une des revendications précédentes, dans lequel le dispositif de documentation (5) est flexible.

11. Système de récipient (1) selon une des revendications précédentes, dans lequel le dispositif de documentation (5) est relié de manière permanente au récipient (3), de préférence par soudure et/ou collage et/ou encastrement.

12. Utilisation d'un système de récipient (1) selon une des revendications précédentes comme récipient de stockage et/ou récipient de réaction dans une application à obligation de documentation.

13. Procédé de fabrication d'un système de récipient (1) comprenant les étapes :
- mise à disposition d'un récipient (3),
- mise à disposition d'un dispositif de documentation électronique (5) avec
-- un dispositif temporisateur (11) pour détecter un instant,
-- au moins un dispositif de capteur (13 ; 15),
-- au moins un dispositif de saisie (21 ; 23 ; 25) qui est conçu pour détecter un actionnement manuel,
-- un dispositif de mémoire (17) qui est conçu pour mémoriser de manière lisible au moins une valeur de mesure détectée par l'au moins un dispositif de capteur (13 ; 15) et/ou au moins une saisie détectée par l'au moins un dispositif de saisie (21 ; 23 ; 25), et
-- au moins un dispositif d'affichage (27 ; 29) pour afficher à un utilisateur un état du dispositif de documentation (5) et/ou un contenu mémorisé dans le dispositif de mémoire,
dans lequel un instant correspondant à leur détection est associé à l'au moins une valeur de mesure et/ou l'au moins une saisie au moyen du dispositif temporisateur (11),
- fixation du dispositif de documentation (5) au récipient (3).

14. Procédé selon la revendication 13, comprenant en outre l'étape :
- réalisation d'une synchronisation temporelle du dispositif temporisateur (11) et/ou
- mémorisation de la date de fabrication et/ou du numéro de charge et/ou de la date d'expiration dans le dispositif de mémoire (17).

15. Procédé selon une des revendications 13 à 14, comprenant en outre l'étape :
- fermeture du récipient (3) au moyen d'un dispositif de scellement (9).
